# EUROPÄISCHE PATENTSCHRIFT

(11) **EP 0 901 467 B1**
(45) Veröffentlichungstag und Bekanntmachung des Hinweises auf die Patenterteilung: **25.07.2001**
(21) Anmeldenummer: 97923841.7
(22) Anmeldetag: 02.05.1997
(51) Int. Cl.: C07C 403/16

(54) **VERBESSERTES VERFAHREN ZUR HERSTELLUNG ON IONONEN, INSBESONDERE VON BETA-IONONEN**
IMPROVED PROCESS FOR PRODUCING IONONES, ESPECIALLY BETA-IONONES
AMELIORATION APPORTEE A UN PROCEDE DE PRODUCTION DE IONONES, NOTAMMENT DE BETA-IONONES

(30) Priorität: 14.05.1996 DE 19619557
(43) Veröffentlichungstag der Anmeldung: 17.03.1999
(73) Patentinhaber: BASF AKTIENGESELLSCHAFT, 67056 Ludwigshafen (DE)
(72) Erfinder: RHEUDE, Udo, D-67166 Otterstadt (DE); HÖRCHER, Ulrich, D-68199 Mannheim (DE); WELLER, Dietmar, D-67067 Ludwigshafen (DE); STROEZEL, Manfred, D-68549 Ilvesheim (DE)
(86) Internationale Anmeldenummer: EP9702249
(87) Internationale Veröffentlichungsnummer: WO9743254

(56) Entgegenhaltungen:
- DE-A- 1 568 108
- DE-A- 4 220 239
- CHEMICAL ABSTRACTS, vol. 081, no. 7, 19.August 1974 Columbus, Ohio, US; abstract no. 037679, KUWATA T ET AL: "Continuous manufacture of methyl ionone in high yields" XP002034701 & JP 49 000 172 - (HASEGAWA, T., CO., LTD.) 5.Januar 1974
- CHEMICAL ABSTRACTS, vol. 083, no. 1, 7.Juli 1975 Columbus, Ohio, US; abstract no. 010527, LEBEDEV I M ET AL: ".beta.-Ionone" XP002034702 & SU 458 540 - (USSR) 30.Januar 1975

## Beschreibung

Die Erfindung betrifft ein verbessertes Verfahren zur kontinuierlichen Herstellung von α- und/oder β-Jonon oder Homologen dieser Verbindungen durch Cyclisieren von Pseudojononen mittels konzentrierter Schwefelsäure in Gegenwart von organischen Lösungs- oder Verdünnungsmitteln und durch Verdünnen des Reaktionsgemisches mit Wasser.

Es ist bekannt, daß bei der Cyclisierung von Pseudojonon in Gegenwart von Säuren wie Schwefelsäure oder Phosphorsäure ein Gemisch von α- und β-Jonon erhalten wird. Das Verhältnis der Mengen, in welchen diese Verbindungen entstehen, ist stark von den Bedingungen, unter denen die Reaktion stattfindet, abhängig.

Da sowohl α-Jonon als auch β-Jonon von großer technischer Bedeutung sind, hat es nicht an Versuchen gefehlt, ein möglichst vorteilhaftes Verfahren zu deren Herstellung zu entwickeln.

Besonders bewährt haben sich Verfahren zur Cyclisierung von Pseudojonon mit konzentrierter Schwefelsäure. Da diese Umsetzung stark exotherm verläuft, ist es sehr wichtig, die entstehende Reaktionswärme möglichst schnell abzuführen, um örtliche Überhitzungen zu vermeiden. Zu diesem Zweck wurden dem Reaktionsgemisch gemäß den bekannten Verfahren Verdünnungsmittel zugegeben. So werden beispielsweise gemäß den DE-PS 10 80 105 und 16 68 505 aliphatische oder cycloaliphatische Kohlenwasserstoffe verwendet. Nachteilig an diesem Verfahren ist, daß sich bei der hier beschriebenen Verfahrensweise in den Reaktionsgefäßen relativ schnell Harze abscheiden, die den kontinuierlichen Betrieb stören.

Gemäß der IN-PS 77 225 wird die Umsetzung in Gegenwart von aliphatischen Chlorkohlenwasserstoffen wie Methylenchlorid, Ethylendichlorid, Chloroform und Tetrachlorkohlenstoff bei Temperaturen von -10°C bis +10°C durchgeführt.

Gemäß der Beschreibung der DE-OS 15 68 108 ist dieses indische Verfahren nachteilig, da die aliphatischen Chlorkohlenwasserstoffe mit Schwefelsäure Chlorwasserstoff abspalten, wodurch die verwendeten Apparate in kurzer Zeit korrodieren. Zur Vermeidung dieser Nachteile wird empfohlen, die Cyclisierung bei -25°C bis +10°C in einem Gemisch aus niedrig-siedenden Kohlenwasserstoffen und Chlorkohlenwasserstoffen durchzuführen. Nachteilig an beiden letztgenannten Verfahren ist, daß man die Reaktionstemperatur mit aufwendigen Kühlmitteln niedrig halten muß, um gute Jonon-Ausbeuten zu erzielen.

Weiterhin sind Verfahren bekannt, bei denen die beträchtliche Cyclisierungswärme durch Siedekühlung mit Flüssiggasen abgeführt wird. So arbeitet man gemäß dem Verfahren der DE-PS 16 68 496 mit flüssigem Schwefeldioxyd, dem Verfahren der DE-PS 16 68 505 mit Propan, Butan oder Isobutan und dem Verfahren der DE-PS 19 17 132 mit Methylchlorid und bei Temperaturen von -25°C bis Raumtemperatur, vorzugsweise Temperaturen unterhalb von +10°C.

Die gemäß diesen Verfahren erzielten Ergebnisse sind im allgemeinen recht gut. Nachteilig heran ist der große Aufwand, der nötig ist, um das bei der Reaktion verdampfte Gas wieder zu verflüssigen.

Aus der CS-PS 179 046, der SU-PS 458 540 und der SU-PS 547 445 sind ferner Verfahren zur Herstellung von β-Jonon bekannt, bei denen eine gute Durchmischung der Reaktanten und eine schnelle Wärmeabfuhr dadurch erreicht werden, daß man einen Dünnfilmreaktor verwendet. Nachteilig an den beiden letztgenannten Verfahren ist, daß man pro m² Dünnfilmfläche und Stunde nur etwa 3 bis 6 kg β-Jonon erhält, und daß somit eine Übertragung in den industriellen Maßstab zu riesigen Apparaturen führen würde. Nachteilig an dem Verfahren des tschechischen Patents ist, daß man zur Erzielung guter Ausbeuten bei Temperaturen zwischen 10 und 15°C arbeiten muß, wodurch wiederum teure Kühlmittel notwendig werden.

Bei allen bekannten Verfahren bildet sich immer ein Gemisch von α- und β-Jonon. Nach den DE-PS 10 80 105, 16 68 496 und 16 68 505 erhält man bei Reaktionstemperaturen von -20 bis O°C bevorzugt β-Jonon, währen bei Temperaturen von -10 bis 25°C der α-Jonon-Gehalt ansteigt.

Weiterhin ist aus EP 133 668 ein Verfahren zur kontinuierlichen Herstellung von Jononen bekannt, bei dem man das Pseudojonon in einem unter den Reaktionsbedingungen bei 25 bis 65°C siedenden Kohlenwasserstoff unter intensiver Durchmischung und Siedekühlung durch teilweises oder vollständiges Verdampfen des Lösungsmittels so mit konzentrierter Schwefelsäure zusammenbringt, daß die Temperatur des Reaktionsgemisches zwischen 25 und 65°C liegt und die Verweilzeit bis zum Verdünnen des Reaktionsgemisches mit Wasser 0,05 bis 20 Sekunden beträgt. Nachteilig an diesem in kleinen Anlagen sehr gut funktionierenden Verfahren ist, daß die Übertragung in großtechnischen Maßstab Schwierigkeiten bereitet.

Schließlich ist aus EP 628 544 A1 ein Verfahren zur Herstellung von β-Ionon durch die mit Schwefelsäure katalysierte Cyclisierung von Pseudojonon in einem aus konzentrierter Schwefelsäure und einem mit Wasser im wesentlichen nicht mischbaren zweiten Lösungsmittel bestehenden zweiphasigen Lösungsmittelsystem bekannt, bei dem als zweites Lösungsmittel unter Druck stehendes flüssiges Kohlendioxid verwendet wird. Nachteilig an diesem Verfahren ist, daß man unter sehr hohen Drucken und bei recht niedrigen Temperaturen arbeiten muß und daß der apparative Aufwand zur Durchführung des Verfahrens doch recht erheblich ist.

β-Jonon ist ein essentielles Vorprodukt für die technische Herstellung von Vitamin A. Ein hoher Gehalt an α-Jonon führt dabei zu einer Verminderung der Ausbeute. Reines α-Jonon und alkylsubstituierte Jonone dagegen sind begehrte Riechstoffe, in welchen ein höherer Gehalt an β-Jonon störend wirken würde.

Es war daher die Aufgabe der Erfindung, ein Verfahren zu entwikkeln, mit dem sowohl möglichst reines α-Jonon als auch möglichst reines β-Jonon oder aber alkylsubstituierte Jonone auch in industriellem Maßstab auf möglichst vorteilhafte Weise in hohen Ausbeuten und Raum-Zeit-Ausbeuten hergestellt werden können.

Gegenstand der Erfindung ist ein sehr vorteilhaftes Verfahren zur Herstellung von Jononen der allgemeinen Formeln I und II 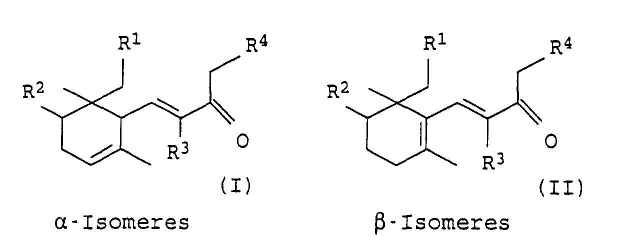 in denen R¹ bis R⁴ für H, -CH₃ oder -C₂H₅ stehen, durch Cyclisieren von Pseudojononen der allgemeinen Formel III 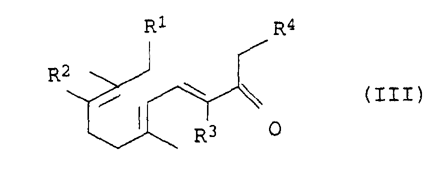 mittels konzentrierter Schwefelsäure bei Temperaturen von 20 bis 90°C, vorzugsweise 35 bis 65°C, insbesondere 40 bis 60°C, in Gegenwart von organischen Lösungs- oder Verdünnungsmitteln unter Kühlung und durch anschließendes Abbrechen der Reaktion durch Hydrolyse des Reaktionsgemisches mit Wasser oder verdünnter Schwefelsäure, dadurch gekennzeichnet, daß man sowohl die Cyclisierung von Pseudojononen der Formel III als auch die anschließende Hydrolyse des Reaktionsgemisches in einer praktisch adiabatischen Reaktion in einer oder mehreren hintereinander geschalteten Reaktionsmischpumpe(n) durchführt, die im wesentlichen aus einer aus einer Umfangswand und zwei Stirnwänden gebildeten rotationssymmetrischen Mischkammer und einem darin drehangetriebenen Mischrotor aus schwefelsäureinertem Material besteht, wobei die Mischkammer mindestens eine Einlaßöffnung für jede Komponente und eine Auslaßöffnung für das Reaktionsgemisch und in den Stirnwänden strömungstechnisch miteinander verbundene ringförmige Kanäle aufweist, und daß man die Reaktionswärme beider Reaktionen jeweils mit Hilfe eines nachgeschalteten Wärmeaustauschers teilweise bzw. ganz abführt.

Eine als sogenannte Reaktionsmischpumpe für das erfindungsgemäße Verfahren geeignete Pump-Misch-Vorrichtung wird beispielsweise in der DE-A-42 20 239 beschrieben, welche durch Zitieren in diese Anmeldungen aufgenommen wird. Verschiedene Ausführungen solcher Reaktionsmischpumpen sind z.B von der Firma K-ENGINEERING, Sessinghausen 26, in 58 566 Kierspe, kommerziell erhältlich. Diese Vorrichtungen vereinigen in sich die Eigenschaften einer Pumpe, eines Mischers zur besonders effektiven Durchmischung sowie des Reaktors und dies sowohl für die Cyclisierung der Pseudojonone der Formel III als auch für die anschließende Hydrolyse des Reaktionsgemisches. Hierdurch bedarf das erfindungsgemäße Verfahren eines sehr geringen apparativen Aufwandes.

Die Mischkammer dieser Reaktionspumpe besteht aus einem Lagerträger und einem zylindrischen Einsatzelement mit einer den Mischrotor über greifenden Umfangswand. In der Umfangswand von Reaktionspumpe(n) 1 befinden sich je eine Einlaßöffnung für die Pseudojononlösung und die konzentrierte Schwefelsäure, sowie eine Auslaßöffnung für das Reaktionsgemisch und in der von Reaktionspumpe(n) 2 je eine Einlaßöffnung für das Reaktionsgemisch und Wasser, bzw. verdünnte Schwefelsäure sowie eine Auslaßöffnung für das Reaktionsgemisch, welches nach Abkühlung an dem nachgeschalteten Wärmeaustauschers in an sich bekannter Weise in die beiden Phasen getrennt und die organische Phase destillativ aufgearbeitet werden kann.

Als besonders günstig hat es sich erwiesen, die Eintrittsöffnungen der Reaktionspumpen in Richtung auf die Reaktionsmischkammer(n) düsenartig verjüngt zu gestalten, weil dadurch eine Art Saugeffekt entsteht.

Selbstverständlich müssen die Teile der Reaktionspumpe die mit dem Reaktionsgemisch in Berührung gelangen aus schwefelsäurestabilem Material gefertigt oder damit verkleidet sein. Als schwefelsäurestabiles Material seien Metalle bzw. Metallegierungen, wie Hastelloy, Titan oder Nickel; Kunstoffe, wie Polyethylen (PE), Polypropylen (PP), Polyvinylidenfluorid (PVDF) oder Polytetrafluorethylen (PFE), oder Oxidkeramik genannt.

Die technische Auslegung der Reaktionspumpen erfolgt in der Regel entsprechend den gewünschten Druckverhältnissen in der Mischkammer.

Im allgemeinen werden sowohl die Cyclisierung der Pseudojonone der Formel III als auch die anschließende Hydrolyse des Reaktionsgemisches bei Drucken von 1 bis 10 bar, vorzugsweise 1,5 bis 2,5 bar durchgeführt.

Die Verweilzeiten der Reaktionsgemische in den Reaktionspumpen werden von den eingestellten Zulaufmengen und der technischen Auslegung der Reaktionspumpen bzw. von der Drehgeschwindigkeit des Mischrotors bestimmt.

Zur Herstellung von α- bzw. β-Jonon durch Cyclisierung von Pseudojonon wird sowohl die Cyclisierung als auch die anschließende Hydrolyse des Reaktionsgemisches mit Verweilzeiten von 0,1 bis 10 Sekunden, vorzugsweise 0,5 bis 2 Sekunden durchgeführt.

Die Cyclisierung von Homologen des Pseudojonons, das heißt Jononen der Formel III, in der mindestens einer der Reste R¹ bis R⁴ für -CH₃ oder -C₂H₅ stehen, erfordert allerdings längere Verweilzeiten. Insbesondere bei der Herstellung von Homologen von α-bzw. β-Ionon kann es daher von Vorteil sein, für jeden Reaktionsschritt jeweils anstelle einer Reaktionspumpe zwei oder mehrere hintereinandergeschaltete Reaktionspumpen zu verwenden.

Die Verweilzeiten betragen beispielsweise für die Herstellung von Methyl-Jononen je nach der Reaktionstemperatur etwa 0,2 bis 20 Sekunden, insbesondere 1 bis 4 Sekunden.

Die als Ausgangsverbindungen verwendeten Pseudojonone sind bekannte Verbindungen, die auf bekannte Weise erhältlich sind.

Die Konzentration der bei der Cyclisierung verwendeten Schwefelsäure kann zwischen 60 und 100 Gew.-% liegen. Vorzugsweise verwendet man 80 bis 98 Gew.-%ige, insbesondere 90 bis 96 Gew.-%ige Schwefelsäure. Pro Mol Pseudojonon verwendet man im allgemeinen 2 bis 10 Mol, vorzugsweise 2 bis 7 Mol, insbesondere 4-6 Mol Schwefelsäure. Bei Verwendung von 2 bis 3 Mol Schwefelsäure pro Mol Pseudojonon erhält man überwiegend α-Jonon, während man beim Einsatz von mehr als 5 Mol Schwefelsäure pro Mol Pseudojonon β-Jonon mit einem Gehalt von weniger als 2 % α-Jonon erhält.

Als Lösungsmittel kommen im wesentlichen aromatische, aliphatische oder cycloaliphatische Kohlenwasserstoffe sowie aliphatische Chlorkohlenwasserstoffe in Betracht. Genannt seien insbesondere Pentan, Hexan, Heptan, Isopentan sowie Cyclohexan oder Gemische hiervon. Von besonderem Vorteil ist es natürlich, solche Lösungsmittel zu verwenden, die unter Normalbedingungen bei Temperaturen zwischen 25 und 100°C sieden. Vorzugsweise verwendet man Hexan (Kp=68,7°C).

Die Lösungsmittelmenge ist in weiten Grenzen variierbar. Die besten Ergebnisse erzielt man jedoch, wenn man mit Lösungen arbeitet, die das Pseudojonon in einer Konzentration von 5 bis 95 Gew.-%, vorzugsweise 10 bis 50 Gew.-%, insbesondere 10 bis 30 Gew.-% enthalten.

Unmittelbar nach Beendigung der Cyclisierung wird das Reaktionsgemisch, das im allgemeinen eine Temperatur von 35 bis 65°C aufweist, in einem Wärmeaustauscher - je nach der Reaktionstemperatur - auf etwa 20 bis 60°C abgekühlt und dann zum endgültigen Abbruch der Reaktion in einer zweiten Reaktionspumpe, oder einer Reihe von Reaktionspumpen, mit Wasser verdünnt. Man verwendet in der Regel 1,5 bis 2,5 l Wasser pro kg Schwefelsäure.

Unmittelbar nach Verlassen der Hydrolyse-Reaktionspumpe(n) wird das Reaktionsgemisch, das im allgemeinen Temperaturen von etwa 35 bis 65°C aufweist, in einem weiteren Wärmeaustauscher auf Temperaturen von etwa 20 bis 60°C, vorzugsweise 30 - 50°C, abgekühlt, dann in einem Phasentrenngefäß die wäßrigeSchwefelsäure-Phase von der erhaltenen Jononlösung abgetrennt und letztere in an sich bekannter Weise destillativ aufgearbeitet.

Mit Hilfe des erfindungsgemäßen Verfahrens können α- und β-Jonon sowie Homologe davon auf technisch sehr einfache und vorteilhafte Weise in hoher Ausbeute und hoher Raumzeitausbeute hergestellt werden. Sowohl hinsichtlich der Anlagenkosten, des Platzbedarfs, des Energieverbrauchs, der Ausbeute und des Personalbedarfs ist das erfindungsgemäße Verfahren hervorragend geeignet.

Die folgenden Beispiele sollen das erfindungsgemäße Verfahren erläutern.

### Beispiele 1-9

In einer Anlage, wie sie in dem beigefügten Verfahrensschema (vgl. Figur) schematisch dargestellt ist, wurde in einem Wärmeaustauscher W1 eine Mischung aus jeweils den aus der folgenden Tabelle (Tab1) ersichtlichen Mengen Pseudojonon (Psi) und Hexan (He) auf die in Tab.1 angegebene Temperatur T1 vorgekühlt und die gekühlte Mischung durch eine Vorrichtung zum Pumpen und Herstellen von Gemischen (im folgenden Reaktionspumpe P1 genannt) bei Drucken zwischen 1 und 10 bar und Verweilzeiten zwischen 0,1 und 10 Sekunden bei den aus Tab.1 ersichtlichen Temperaturen T 2 mit konzentrierter Schwefelsäure (Ss) der aus Tab.1 ersichtlichen Konzentration eingepumpt und intensiv vermischt und dadurch das Pseudojonon zu einem Gemisch der aus Tab.1 ersichtlichen Jonone umgesetzt. Sofort nach Verlassen der Reaktionspumpe 1 wurde ein Teil der freigesetzten Wärme durch Abkühlen an dem Wärmetauscher W2 auf etwa 40°C (vgl. T3 in Tab.1) abgeführt. Anschließend wurde das Reaktionsgemisch in eine zweite Vorrichtung zum Pumpen und Herstellen von Gemischen (im folgenden Reaktionspumpe P2 genannt) gesaugt, wo es bei Drucken von 1 bis 10 bar und Verweilzeiten zwischen 0,1 und 10 Sekunden unter sehr gutem Vermischen mit vollentsalztem Wasser (veW) versetzt wurde. In einem nachgeschalteten Wärmetauscher W3 wurde die aufgetretene Reaktionswärme der Hydrolyse durch Abkühlen auf etwa 40°C weitgehend abgeführt. In einem nachgeschalteten Phasentrenngefäß wurde die verdünnte Schwefelsäure von den erhaltenen Jononen abgetrennt.

In Tab.1 sind die im Rohaustrag enthaltenen Mengen an α- und β-Jonon (angegeben in Flächenprozenten, ermittelt durch Gaschromatographie) angegeben.

| Beispiele | | | | | | | | | |
|---|---|---|---|---|---|---|---|---|---|
| | 1 | 2 | 3 | 4 | 5 | 6 | 7 | 8 | 9 |
| Pseudojonon-Zulaufmenge [kg/h] | 135 | 270 | 260 | 150 | 200 | 135 | 300 | 135 | 135 |
| Hexan-Zulaufmenge [kg/h] | 800 | 800 | 800 | 800 | 800 | 800 | 800 | 800 | 800 |
| Schwefelsäure-Zulaufmenge [kg/h] | 370 | 740 | 710 | 410 | 550 | 340 | 820 | 380 | 400 |
| Schwefelsäure-Gehalt [Gew.-%] | 93 | 93 | 95 | 95 | 95 | 98 | 95 | 91 | 84 |
| Hydrolysewasser-Zulaufmenge [kg/h] | 765 | 1500 | 1130 | 700 | 1000 | 765 | 1050 | 820 | 820 |
| | | | | | | | | | |
| Betajonon-Gehalt im Rohaustrag [GC-F1.-%] | 90,7 | 93,0 | 93,4 | 93,5 | 91,8 | 92,2 | 93,5 | 87,3 | 57,3 |
| Alphajonon-Gehalt im Rohaustrag [GC-F1.-%] | 3,0 | 1,9 | 1,2 | 1,4 | 1,5 | 0,6 | 1,2 | 5,2 | 36,1 |
| Betajonon-Ausbeute [%] | 81,0 | 82,0 | 85,5 | 84,7 | 81,9 | 77,5 | 82,6 | 79,5 | n.b. |
| | | | | | | | | | |
| Temperatur T1 [°C] | 10 | -2 | -3 | 12 | 5 | 5 | -7 | 10 | 10 |
| Temperatur T2 [°C] | 47 | 54 | 59 | 50 | 59 | 52 | 59 | 49 | 42 |
| Temperatur T3 [°C] | 40 | 40 | 41 | 40 | 41 | 39 | 44 | 46 | 40 |
| Temperatur T4 [°C] | 52 | 52 | 61 | 57 | 58 | 58 | 64 | 47 | 42 |
| Temperatur T5 [°C] | 40 | 40 | 40 | 40 | 40 | 40 | 40 | 40 | 40 |

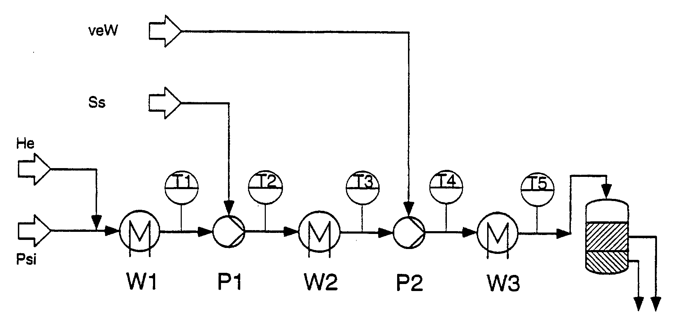

## Patentansprüche

1. Verfahren zur kontinuierlichen Herstellung von Jononen der allgemeinen Formeln I und II 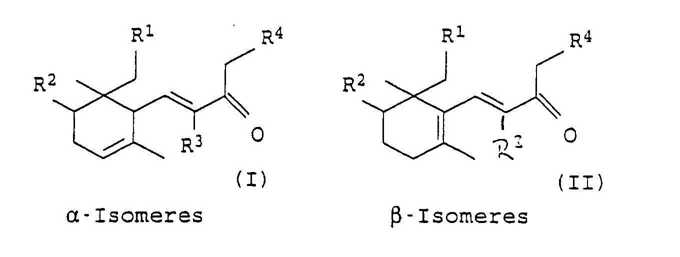 in denen R¹ bis R⁴ für H, -CH₃ oder -C₂H₅ stehen, durch Cyclisieren von Pseudojononen der allgemeinen Formel III 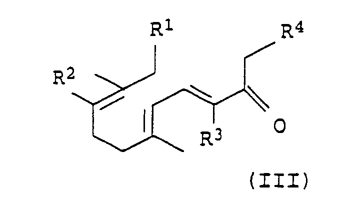 mittels konzentrierter Schwefelsäure bei Temperaturen von 20 bis 90°C in Gegenwart von organischen Lösungs- oder Verdünnungsmitteln unter Kühlung und durch anschließendes Abbrechen der Reaktion durch Hydrolyse des Reaktionsgemisches mit Wasser, dadurch gekennzeichnet, daß man sowohl die Cyclisierung von Pseudojononen der Formel III als auch die anschließende Hydrolyse des Reaktionsgemisches in einer praktisch adiabatischen Reaktion in einer oder mehreren hintereinander geschalteten Reaktionsmischpumpe(n) durchführt, die im wesentlichen aus einer aus einer Umfangswand und zwei Stirnwänden gebildeten rotationssymmetrischen Mischkammer und einem darin drehangetriebenen Mischrotor aus schwefelsäureinertem Material besteht,

2. Verfahren gemäß Anspruch 1, dadurch gekennzeichnet, daß die Cyclisierung der Pseudojonone und die anschließende Hydrolyse des Reaktionsgemisches bei Drucken von 1 bis 10 bar, vorzugsweise 1,5 bis 2,5 bar durchgeführt wird.

3. Verfahren gemäß Anspruch 1, dadurch gekennzeichnet, daß man zur Herstellung von α-bzw. β-Ionon die Cyclisierung von Pseudojonon und die anschließende Hydrolyse des Reaktionsgemisches mit Verweilzeiten von 0,1 bis 10 Sekunden, vorzugsweise 0,5 bis 2 Sekunden durchführt.

4. Verfahren gemäß Anspruch 1, dadurch gekennzeichnet, daß man die Cyclisierung der Pseudojonone der Formel III und die Hydrolyse des Reaktionsgemisches in Gegenwart von einem geeigneten aromatischen, aliphatischen oder cycloaliphatischen Kohlenwasserstoff oder einem aliphatischen Chlorkohlenwasserstoff oder aber einem Gemisch der genannten Kohlenwasserstoffe oder Chlorkohlenwasserstoffe als Lösungs- oder Verdünnungsmittel durchführt.

5. Verfahren gemäß Anspruch 1, dadurch gekennzeichnet, daß man die Cyclisierung der Pseudojonone der Formel III und die Hydrolyse des Reaktionsgemisches in Gegenwart von Pentan, Hexan, Heptan oder Gemischen hiervon als Lösungs- oder Verdünnungsmittel durchführt.

6. Verfahren gemäß Anspruch 1, dadurch gekennzeichnet, daß man sowohl die Cyclisierung der Pseudojonone der Formel III als auch die anschließende Hydrolyse des Reaktionsgemisches bei Temperaturen von 35 bis 65°C durchführt.

7. Verfahren gemäß Anspruch 1, dadurch gekennzeichnet, daß man die Cyclisierung der Pseudojonone der Formel III mit Schwefelsäure einer Konzentration von 60 bis 100 Gew.-% durchführt.

8. Verfahren gemäß Anspruch 1, dadurch gekennzeichnet, daß man die Cyclisierung der Pseudojonone der Formel III mit Lösungen der Pseudojonone in den Lösungs- oder Verdünnungsmitteln durchführt, in denen die Konzentration der Pseudojonone 5 bis 95 Gew.-% beträgt.

## Claims

1. A process for the continuous preparation of ionones of the general formulae I and II 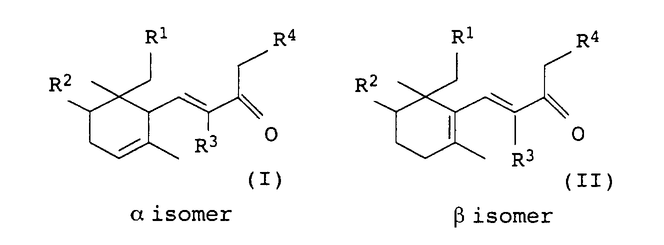 in which R¹ to R⁴ are H, -CH₃ or -C₂H₅, by cyclizing pseudoionones of the general formula III 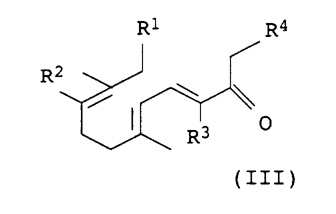 using concentrated sulfuric acid at temperatures from 20 to 90°C in the presence of organic solvents or diluents with cooling and by subsequent termination of the reaction by hydrolysis of the reaction mixture with water, wherein both the cyclization of pseudoionones of the formula III and the subsequent hydrolysis of the reaction mixture are carried out in a virtually adiabatic reaction in one or more reaction mixing pump(s) which are connected in series and each of which essentially comprises a rotationally symmetrical mixing chamber formed from a peripheral wall and two end walls and of a mixing rotor made of material inert to sulfuric acid and with rotational drive.

2. A process as claimed in claim 1, wherein the cyclization of the pseudoionones and subsequent hydrolysis of the reaction mixture are carried out under pressures from 1 to 10 bar, preferably 1.5 to 2.5 bar.

3. A process as claimed in claim 1, wherein to prepare α- or β-ionone the cyclization of pseudoionone and the subsequent hydrolysis of the reaction mixture are carried out with residence times of from 0.1 to 10 seconds, preferably 0.5 to 2 seconds.

4. A process as claimed in claim 1, wherein the cyclization of the pseudoionones of the formula III and the hydrolysis of the reaction mixture are carried out in the presence of a suitable aromatic, aliphatic or cycloaliphatic hydrocarbon or an aliphatic chlorinated hydrocarbon or else a mixture of said hydrocarbons or chlorinated hydrocarbons as solvents or diluents.

5. A process as claimed in claim 1, wherein the cyclization of the pseudoionones of the formula III and the hydrolysis of the reaction mixture are carried out in the presence of pentane, hexane, heptane or mixtures thereof as solvents or diluents.

6. A process as claimed in claim 1, wherein both the cyclization of the pseudoionones of the formula III and the subsequent hydrolysis of the reaction mixture are carried out at temperatures from 35 to 65°C.

7. A process as claimed in claim 1, wherein the cyclization of the pseudoionones of the formula III is carried out with sulfuric acid of a concentration of 60 to 100% by weight.

8. A process as claimed in claim 1, wherein the cyclization of the pseudoionones of the formula III is carried out with solutions of the pseudoionones in the solvents or diluents in which the concentration of the pseudoionones is 5 to 95% by weight.

## Revendications

1. Procédé pour la préparation continue des ionones de formules générales I et II 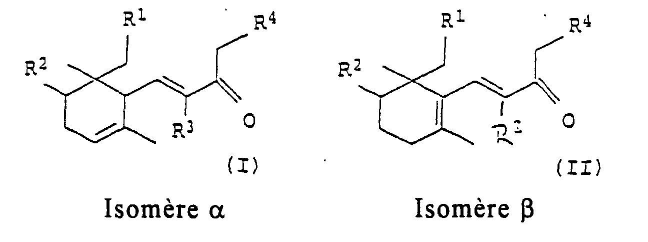 dans lesquelles R¹ à R⁴ représentent H, -CH₃ ou -C₂H₅, par cyclisation de pseudo-ionones de formule générale III 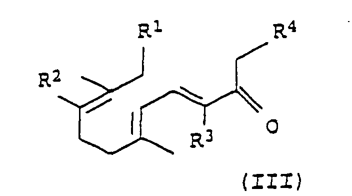 à l'aide de l'acide sulfurique concentré, à des températures de 20 à 90°C, en présence de solvants ou diluants organiques, sous refroidissement et en arrêtant ensuite la réaction par hydrolyse du mélange de réaction à l'aide d'eau, caractérisé par le fait que l'on procède à la cyclisation des pseudo-ionones de formule III comme à l'hydrolyse subséquente du mélange de réaction dans une réaction pratiquement adiabatique dans une ou plusieurs pompes mélangeuses de réaction disposées les uns derrière les autres, qui consistent essentiellement en une chambre de mélange symétrique à l'égard de la rotation, constituée d'une paroi périphérique et de deux parois frontales avec, à l'intérieur de cette chambre de mélange, un rotor mélangeur en matériau inerte à l'égard de l'acide sulfurique soumis à rotation.

2. Procédé selon la revendication 1, caractérisé par le fait que la cyclisation des pseudo-ionones et l'hydrolyse subséquente du mélange de réaction sont exécutées sous des pressions de 1 à 10 bar, de préférence de 1,5 à 2,5 bar.

3. Procédé selon la revendication 1, caractérisé par le fait que, pour la préparation de l'alpha- ou de la bêta-ionone, la cyclisation des pseudo-ionones et l'hydrolyse subséquente du mélange de réaction sont exécutées avec des durées de passage de 0,1 à 10 s, de préférence de 0,5 à 2 s.

4. Procédé selon la revendication 1, caractérisé par le fait que la cyclisation des pseudo-ionones de formule III et l'hydrolyse du mélange de réaction sont exécutées en présence d'un hydrocarbure aromatique, aliphatique ou cycloaliphatique approprié ou d'un hydrocarbure aliphatique chloré ou encore d'un mélange desdits hydrocarbures ou hydrocarbures chlorés, servant de solvant ou diluant.

5. Procédé selon la revendication 1, caractérisé par le fait que la cyclisation des pseudo-ionones de formule III et l'hydrolyse du mélange de réaction sont exécutées en présence de pentane, d'hexane, d'heptane ou leurs mélanges, servant de solvants ou diluants.

6. Procédé selon la revendication 1, caractérisé par le fait que la cyclisation des pseudo-ionones de formule III comme l'hydrolyse subséquente du mélange de réaction sont réalisées à des températures de 35 à 65°C.

7. Procédé selon la revendication 1, caractérisé par le fait que la cyclisation des pseudo-ionones de formule III est réalisée à l'aide d'acide sulfurique à une concentration de 60 à 100 % en poids.

8. Procédé selon la revendication 1, caractérisé par le fait que la cyclisation des pseudo-ionones de formule III est exécutée sur des solutions des pseudo-ionones dans les solvants ou diluants, à une concentration de 5 à 95 % en poids des pseudo-ionones.
